# EUROPEAN PATENT SPECIFICATION

(11) **EP 4 633 585 B1**
(45) Date of publication and mention of the grant of the patent: **26.08.2026**
(21) Application number: 23832747.2
(22) Date of filing: 14.12.2023
(51) Int. Cl.: A61K 8/58, A61K 8/73, A61K 31/167, A61K 31/695, A61K 31/728, A61P 17/00, A61Q 19/08

(54) **COMBINATION OF AN ORGANOSILICON COMPOUND WITH A CROSSLINKED GLYCOSAMINOGLUCAN FOR PREVENTING AND REPAIRING CUTANEOUS AGEING**
KOMBINATION EINER ORGANOSILICIUMVERBINDUNG MIT EINEM VERNETZTEN GLYCOSAMINOGLUCAN ZUR VORBEUGUNG UND REPARATUR VON HAUTALTERUNG
COMBINAISON D'UN COMPOSÉ ORGANOSILICIÉ ET D'UN GLYCOSAMINOGLUCANE RÉTICULÉ POUR PRÉVENIR ET RÉPARER LE VIEILLISSEMENT CUTANÉ

(30) Priority: 16.12.2022 EP 22214065
(43) Date of publication of application: 22.10.2025
(73) Proprietor: Laboratoires Fill-Med, 75008 Paris (FR)
(72) Inventor: IZZO, Elisabetta, 1050 Ixelles (BE); SENTIER, Luc, 1620 Drogenbos (BE)
(74) Representative: Bandpay & Greuter
(86) International application number: PCT/EP2023/085914
(87) International publication number: WO 2024/126728

(56) References cited:
- EP-B1- 1 750 770
- US-B2- 8 822 433

## Description

### Technical Field

The present invention relates to a composition comprising an organosilicon compound and a crosslinked glycosaminoglycan and the use thereof in cosmetic and/or dermatological applications, such as to constrain the organization of protein and glycoprotein fibers of the extracellular matrix in order to prevent and repair cutaneous ageing, in particular in order to increase the native production of elastin and collagen by the skin.

### Background of the invention

At present, there is a great need to develop products that make it possible to improve a pathological condition by mechanical action alone, i.e. without direct pharmacological action.

This type of mode of action is particularly interesting insofar as we are looking for modes of treatment that are as little aggressive as possible for the organism.

There are various techniques for the correction of connective tissue damage or alterations.

For the skin for example, there are numerous products commonly called "fillers", since they act by "filling" the lost volume of the skin.

However, for injectable substances of natural origin, in addition to classical allergy problems, a disadvantage of their use, especially in cosmetic dermatology, is their limited activity time, as they are dependent on tissue catabolism. Indeed, their kinetics show a strong filling effect of depressed volumes post-injection, which does not last long and justifies the need to frequently renew the treatment.

Other techniques of treatment of damaged dermis include mesotherapy / biorevitalization used for skin rejuvenation.

Compounds classically used in skin rejuvenation mesotherapy / biorevitalization include non-crosslinked hyaluronic acid (HA) or organosilicon compounds, as main ingredients and amino acids, vitamins, flavonoids, plant extracts, and others as secondary ingredients.

In particular, silanol has been used for decades in the treatment of skin photoaging as it stabilizes and maintains skin structures through hydrogen bonding electrostatic interaction with extracellular matrix (ECM) proteins or glycosaminoglycans.

Recently, methylsilanol mannuronate (as a source of organic silicon), used in combination with resveratrol, has been reported as promoting the expression of hyaluronan synthase type 2 (HAS2), the enzyme responsible for the production of high-molecular chain hyaluronic acid but also collagen type I and elastin genes in human skin fibroblasts (Deglesne et al., Medical Devices: Evidence and Research 2018:11,313-320). However, this study does not enable to determine which one of the involved active substances, namely methylsilanol mannuronate or resveratrol, is the main fibroblast activator in vitro.

Document WO 2005/082421 discloses the use of an organosilicon compound stabilized by an alcohol, phenol, acid, amine, aminoacid compound, for the manufacture of a preparation for constraining the organization of protein and glycoprotein fibers of the extra-cellular matrix of dermis.

Document US 8,822,433 discloses a complex based on an organic silicon derivative which is combined, by weak bonds, with one or several hyaluronic acid calibrated fragments of molecular weight comprised between 150 and 750 kDa.

In any case, as for fillers, the effect of mesotherapy products does not last long so that it is generally needed to frequently renew the treatment.

Thus, there is a need for alternative compositions which make it possible to remedy the disadvantages mentioned above.

### Summary of the invention

Unexpectedly, it has now been discovered that by introducing an organosilicon compound in combination with a crosslinked glycosaminoglycan *in situ,* in supra-physiological concentrations into the dermis, it is possible to obtain a structural organization of the protein and glycoprotein fibers of the extracellular matrix.

More specifically, it has been discovered that such a combination synergistically stimulates the biosynthesis of both elastin and collagen by human skin fibroblasts. In particular, without wishing to be bound to any particular theory, the crosslinked glycosaminoglycan would prolong the effect of the organosilicon compound over time.

As a further advantage, it has been discovered that the produced collagen is homogeneously distributed.

Thus, in a first aspect, the invention relates to a composition comprising:
- an organosilicon compound of formula (I):

   RₓSi(OH)₄₋ₓ (I)

   or a salt thereof, wherein each R is independently a group containing from 1 to 20 carbon atoms, x = 1 to 3, and
- a crosslinked glycosaminoglycan or a salt thereof.

In some variations, the composition is in the form of an injectable solution.

In some variations, the composition has a pH from 5.0 to 7.0, preferably from 5.5 to 6.5.

In some variations, the glycosaminoglycan is selected from hyaluronic acid or a salt thereof, chondroitin sulfate and keratan sulfate, and is preferably hyaluronic acid or sodium hyaluronate.

In some variations, the molecular weight of the glycosaminoglycan prior to crosslinking ranges from 0.8 to 4.0 MDa, in particular from 1.0 to 2.0 MDa.

In some variations, the organosilicon compound is monomethylsilanetriol or dimethylsilanediol or a salt thereof.

In some variations, the organosilicon compound of formula (I) is present in an amount from 0.1% to 5%, in particular from 0.2% to 1%, by weight, relative to the total weight of the composition.

In some variations, the crosslinked glycosaminoglycan is present in an amount from about 0.5% to about 5.0%, in particular from about 1.5% to about 2.5%, by weight, relative to the total weight of the composition.

In some variations, the weight ratio of the organosilicon compound relative to the crosslinked glycosaminoglycan ranges from 1 to 50%, in particular from 5 to 20%.

In some variations, the composition further comprises an anesthetic agent, preferably lidocaine, or a salt thereof.

In some variations, the anesthetic agent is present in an amount from about 0.1% to about 5%, in particular from about 0.2% to about 0.4%, by weight, relative to the total weight of the composition.

In some variations, the composition is a cosmetic composition.

The invention further relates to a method of cosmetic treatment of the skin, preferably for treating wrinkles or other dermal depressions and/or for improving the condition of the skin, notably for reducing signs of aging, the method comprising a step of administering the above-described composition to a subject.

The invention further relates to the composition as described above, which is a medicament for use in the *in situ* treatment of damaged dermis.

In some variations, a dose comprised between 0.05 and 1.0 mg of said organosilicon compound is administered *in situ* per cm² of treated dermis.

### Detailed description

The invention is now described in more detail and in a non-limiting manner in the following description.

All concentrations are expressed by weight unless otherwise specified.

### Organosilicon compound

The composition of the invention includes an organosilicon compound of formula (I) : RₓSi(OH)₄₋ₓ, or a salt thereof, wherein each R is independently a group containing from 1 to 20 carbon atoms and x = 1 to 3.

In preferred variations, x=1 or 2, and the organosilicon compound is a diol or a triol.

Each R may contain only carbon or hydrogen, or may optionally contain one or more heteroatoms, such as oxygen, sulfur, or nitrogen. Preferably, R does not contain silicon.

Each R may be have a linear, branched or cyclic structure.

Preferably, each R contains from 1 to 10 carbon atoms.

Each R may be notably selected from the following groups: alkyl, substituted alkyl, alkenyl group, substituted alkenyl, phenyl, substituted phenyl, aryl, substituted aryl, heteroaryl, substituted heteroaryl, alkoxy, substituted alkoxy, phenoxy, substituted phenoxy, aroxy, substituted aroxy, alkylthio, substituted alkylthio, phenylthio, substituted phenylthio, arylthio, substituted arylthio, cyano, isocyano, substituted isocyano, carbonyl, substituted carbonyl, carboxyl, substituted carboxyl, amino, substituted amino, amido, substituted amido, sulfonyl, substituted sulfonyl, sulfonic acid, phosphoryl, substituted phosphoryl, phosphonyl, substituted phosphonyl, polyaryl, substituted polyaryl, C3-C20 cyclic, substituted C3-C20 cyclic, heterocyclic, substituted heterocyclic, aminoacid, peptide, and polypeptide groups.

In preferred variations, each R is independently selected from (C1-C4)alkyl, (C2-C6)alkenyl, (C6-C10)aryl, (C1-C6)alkyl-(C6-C10)aryl.

In preferred variations, each R is independently selected from (C1-C4)alkyl, preferably from (C1-C3)alkyl, more preferably from (C1-C2)alkyl, even more preferably is methyl.

When more than one R is present, they may be identical or different.

Examples of preferred organosilicon compounds are monomethylsilanetriol and dimethylsilanediol.

The organosilicon compound may be introduced in the composition in the form of a salt, such as for example a salicylate, mannuronate or alginate.

In some embodiments, more than one organosilicon compound may be present in the composition of the invention. In other, preferred, embodiments, a single organosilicon compound may be present in the composition of the invention.

### Glycosaminoglycan

The composition of the invention includes a glycosaminoglycan, i.e. a polysaccharide comprising repeating disaccharide units, each disaccharide unit consisting of an amino sugar and a non-amino sugar.

The non-amino sugar may be in particular a uronic sugar or galactose.

The amino sugar may be in particular N-acetyl-glucosamine.

The glycosaminoglycan may be in the form of a salt or not.

Glycosaminoglycans include, in particular, heparin, heparan sulfate, chondroitin sulfate, dermatan sulfate, keratan sulfate, hyaluronic acid.

Preferred is hyaluronic acid (also known as hyaluronan) or a salt thereof, such as sodium hyaluronate.

The glycosaminoglycan used in the composition is cross
linked. A crosslinked glycosaminoglycan comprises bonds within linear chains of glycosaminoglycan and/or between linear chains of glycosaminoglycan.

Glycosaminoglycans, in particular hyaluronic acid, can be crosslinked using a crosslinking agent such as for example 1,4-butanediol diglycidyl ether, divinylsulfone, polyethylene glycol or glutaraldehyde. 1,4-butanediol diglycidyl ether is preferred.

An exemplary method of preparing the crosslinked glycosaminoglycan is the following.

Glycosaminoglycan (e.g., hyaluronic acid or sodium hyaluronate) may be hydrated in an aqueous solution and the resulting gel may be mixed or kneaded to produce a substantially homogenous and transparent gel. The concentration of glycosaminoglycan in the gel at this stage may be from 2 to 40% by weight, preferably from 10 to 30% by weight, more preferably from 15 to 25% by weight. A concentration of 20% by weight may in particular be used. Hydration may be performed for instance for 1 to 100 hours, such as for 15 to 60 hours. Mixing or kneading may be performed for instance for 1 minute to 2 hours, preferably for 5 minutes to 1 hour, more preferably for 10 to 30 minutes, such as for approximately 15 minutes.

A base, such as NaOH, may be added to adjust the pH, preferably to a value of at least 11. The concentration of glycosaminoglycan in the gel may then decrease to between 1 and 30% by weight, preferably between 5 and 20% by weight, more preferably between 10 and 15% by weight. A concentration of 12% by weight may in particular be used.

The crosslinking agent (e.g., 1,4-butanediol diglycidyl ether) may then added, for example in an amount of 6 to 10% relative to the weight of glycosaminoglycan. The gel may be further mixed or kneaded, for example for a duration of 5 minutes to 3 hours, preferably 15 minutes to 2 hours, more preferably 30 minutes to 1 hour, such as for approximately 45 minutes.

The gel may then be heated to a temperature for instance from 40 to 55°C, preferably from 45 to 50°C, for a duration which may for instance range from 1 to 12 hours, preferably from 3 to 4.5 hours. The resulting gel may then be cut into pieces.

After being cut into pieces, the gel may be diluted in an aqueous solution, preferably an acidic solution, in order to achieve a pH of from 5 to 9, preferably from 6 to 8. Hydrochloric acid may be used for instance as an acid, in order to neutralize the base used in previous steps of the preparation of the gel. The gel may be brought to a final concentration in glycosaminoglycan of from 0.1 to 10% by weight, preferably from 0.5 to 7.5% by weight, more preferably from 2 to 5% by weight. A final concentration of approximately 3 to 3.5% by weight may in particular be selected. At this stage, the gel may be further mixed or kneaded for a duration which may for example be from 2 to 60 hours, preferably from 10 to 40 hours, more preferably from 18 to 24 hours. Orbital mixing may in particular be used.

The gel may then be purified. For example, a dialysis step may be carried out. The gel may be placed in a dialysis membrane (having a cutoff of e.g. 14 kDa) in a tank containing a buffer, such as physiological phosphate buffer. The buffer may be periodically or continuously renewed. Agitation may be provided. The dialysis may last from 2 to 100 hours, for example from 24 to 36 hours, such as approximately 48 hours. The purification step makes it possible to eliminate low molecular contaminants, such as in particular unreacted crosslinking agent.

The molecular weight of the glycosaminoglycan (in particular hyaluronic acid) prior to crosslinking may range from 0.8 to 4.0 MDa, in particular from 1.0 to 2.0 MDa, for example from 1.1 to 1.4 MDa.

By *"molecular weight"* is meant herein the weight-average molecular weight Mw.

In some embodiments, more than one glycosaminoglycan may be present in the composition of the invention. In other, preferred, embodiments, a glycosaminoglycan may be present in the composition of the invention, preferably hyaluronic acid or sodium hyaluronate.

### Additives

The composition of the invention may comprise one or more additives, which are preferably added to the formulation after the step of purification.

In particular, it may comprise an anesthetic agent.

The anesthetic agent may be selected from ambucaine, amolanone, amylocaine, articaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquine, dimethocaine, diperodone, dycyclonine, ecgonidine, ecgonine, ethylchloride, etidocaine, beta-eucaine, euprocine, fenalcomine, formocaine, hexylcaine, hydroxytetracaine, isobutyl p-aminobenzoate, mesylate leucinocaine, levoxadrol, lidocaine, mepivacaine, meprylcaine, metabutoxycaine, methylchloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, pseudococaine, pyrrocaine, ropivacaine, salicylic alcohol, tetracaine, tolycaine, trimecaine, zolamine, a salt thereof, and combinations thereof. Lidocaine is a preferred anesthetic agent.

The composition of the invention may comprise free hyaluronic acid.

Free hyaluronic acid may have a molecular weight ranging from 0.8 to 4.0 MDa, in particular from 1.0 to 2.0 MDa, for example from 1.1 to 1.4 MDa.

The composition of the invention may comprise the free hyaluronic acid in an amount from 5% to 30%, in particular from 10% to 25%, by weight, relative to the total weight of the initial glycosaminoglycan used for crosslinking. Exemplary weight ranges are: from 5 to 10%, from 10 to 15%, from 15 to 20%, from 20 to 25%.

### Composition

The composition of the invention may comprise the organosilicon compound of formula (I) in an amount from 0.1% to 5%, in particular from 0.2% to 1%, by weight, relative to the total weight of the composition. Exemplary weight ranges are: from 0.1 to 0.2%, from 0.2 to 0.5%, from 0.5 to 1%, from 1 to 2%, from 2 to 5%.

The composition of the invention may comprise the crosslinked glycosaminoglycan in an amount from about 0.5% to about 5.0%, in particular from about 1.5% to about 2.5%, by weight, relative to the total weight of the composition. Exemplary weight ranges are: from 0.5 to 1%, from 1 to 1.5%, from 1.5 to 2%, from 2 to 3%, from 3 to 5%.

The weight ratio of the organosilicon compound relative to the crosslinked glycosaminoglycan in the composition may range from 1 to 50 %, in particular from 5 to 20 %. Exemplary ranges for this weight ratio are: from 1 to 2%, from 2 to 5%, from 5 to 10%, from 10 to 15%, from 15 to 20%, from 20 to 25%, from 25 to 30%, from 30 to 40%, from 40 to 50%.

If an anesthetic agent is present in the composition, it may be in an amount from about 0.1% to about 5%, in particular from about 0.2% to about 0.4%, by weight, relative to the total weight of the composition. Exemplary weight ranges are: from 0.1 to 0.2%, from 0.2 to 0.5%, from 0.5 to 1%, from 1 to 2%, from 2 to 5%.

The composition is preferably in the form of an aqueous solution, more preferably in the form of an injectable solution. The notion of aqueous solution encompasses an aqueous gel, in the present application.

The composition preferably has a pH from 5.0 to 7.0, more preferably from 5.5 to 6.5. These ranges of pH are advantageous in order to prevent the polymerization and precipitation of the organosilicon compound.

The composition of the invention may be prepared by placing the glycosaminoglycan, previously formulated in the form of a gel as described above, in a container preferably equipped with an agitator.

The following ingredients may then be added to the gel, in any order:
- a concentrated solution of organosilicon compound;
- an acidic aqueous solution;
- a solution of anesthetic agent, if present;
- a buffer.

The concentrated solution of organosilicon compound may be a basic solution, for example a sodium hydroxide solution. The concentration of organosilicon compound in this solution may range from 10 to 60%, preferably from 20 to 50%, more preferably from 35 to 45%, by weight. The amount of concentrated solution of organosilicon compound to be added is selected as a function of the desired final concentration of organosilicon compound.

The acidic aqueous solution may be for example a hydrochloric acid solution, e.g. at a concentration of 1M. The amount of acidic aqueous solution to be added is selected to adjust the pH to a desired range.

The solution of anesthetic agent may comprise from 5 to 50% anesthetic agent, preferably from 10 to 30%, more preferably from 15 to 25%, by weight. The solution may be for example a phosphate buffer. The amount of solution of anesthetic agent to be added is selected as a function of the desired final concentration of anesthetic agent.

The buffer may be physiological phosphate buffer. The amount of buffer to be added is selected as a function of the desired final concentration of glycosaminoglycan.

Once all the ingredients have been combined and mixed, the composition may be degassed under agitation. The composition may be packaged. For example, the composition may be transferred to an injection device. The injection device containing the composition may be selected from a syringe, a set of micro-syringes, an injection gun, a needleless injection device, and a microneedle roll.

A kit may be provided, comprising an injection device and one or more containers containing the composition. Each container may contain e.g. from 1 to 5 mL, preferably from 2 to 4 mL of the composition. The containers may be syringes or syringe ampules.

### Use of the composition

The composition can be used for cosmetic and/or dermatological (therapeutic) purposes.

The composition may be administered topically, or preferably by injection. It may be an intraepidermal and/or intradermal and/or subcutaneous injection. Preferably, it is an intradermal injection.

Preferably, the composition according to the invention is administered locally, in the region of the face, in particular of the nasolabial folds, of the periorbital zone, of the contour of the lips, of the forehead and of the area between the eyebrows, in the region of the neck and/or in the neckline area.

The composition may be applied by mesotherapy. By *"mesotherapy"* is meant local and very superficial (intraepidermal, intradermal or hypodermic/subcutaneous) injections of low doses of the composition, at the site where the disorder or pain is felt.

The composition according to the invention may be injected once or several times. The frequency of administration may be constant or may vary. The frequency of administration may be from 10 days to 12 months, preferably from 1 to 12 months, and even more preferably from 1 to 6 months.

In each administration, a dose of composition amounting to between 0.05 and 1.0 mg of the organosilicon compound per cm² of treated dermis may be used.

The composition can be used in particular to treat wrinkles, fine lines, fibroblast depletions and scars; or to prevent wrinkles, fine lines and fibroblast depletions.

In particular, the composition makes it possible to fill in wrinkles and other depressions.

The composition makes it possible to promote cell renewal and thus the synthesis of constituents newly synthesized by fibroblasts, in particular collagen and elastin.

The composition makes it possible to remodel and/or revitalize the dermis.

The composition makes it possible to hydrate the skin.

The composition makes it possible to improve the condition of the skin.

The composition makes to reduce or prevent signs of skin aging, especially UV-induced skin aging.

The invention thus relates to a method of cosmetic treatment comprising a step of administering the composition to a subject, preferably by injection as described above.

In other embodiments, a method of therapeutic, in particular dermatological, treatment is provided, comprising a step of administering the composition to a patient in need thereof, preferably by injection as described above.

The composition can thus be used, as indicated above, for the treatment or prevention of various dermatological conditions, in particular erythema, eczema and neurodermatitis.

Erythema may be of the following types: *erythema ab igne, erythema chronicum migrans, erythema induratum, erythema infectiosum, erythema marginatum, erythema migrans, erythema multiforme, erythema nodosum, erythema toxicum, erythema elevatum diutinum, erythema gyratum repens,* keratolytic winter erythema and palmar erythema.

Eczema may be atopic dermatitis, dyshidrosis, hand eczema, nummular eczema, seborrheic dermatitis, stasis dermatitis, discoid eczema, pompholyx, asteatotic eczema, varicose eczema, allergic eczema (including contact dermatitis, allergic dermatitis and allergic contact dermatitis).

### Examples

The following examples illustrate the invention without limiting it.

In the examples, the following commercial injectable solutions were used by way of comparison:
- ARTFiller Universal (designated as AFU below) marketed by FillMed, comprising crosslinked sodium hyaluronate having a molecular weight of 1.2 MDa and a concentration of 2.5 % by weight, and further containing 0.3 % by weight of lidocaine hydrochloride as an anesthetic agent;
- Conjonctyl (designated as Si below), previously marketed by Exsymol, consisting of monomethylsilanetriol orthohydroxybenzoate at a concentration of 0.055% (equivalent amount of silicon);
- Architect MHA30 (designated as MHA30 below) prepared by FillMed, comprising non-crosslinked (i.e. free) sodium hyaluronate having a molecular weight of 1.2 MDa and a concentration of 3.0 % by weight, and further containing 0.3 % by weight of lidocaine hydrochloride as an anesthetic agent; enriched with an organosilicon compound, namely monomethylsilanetriol salicylate at a final concentration of 0.055% (equivalent amount of silicon).

A composition according to the invention (designated as AFU+Si below) was made by enriching the AFU composition with an organosilicon compound, namely monomethylsilanetriol salicylate. The manufacturing process is the same as for ARTFiller Universal, with the addition of organic silicon in the final formulation, at the same time of additives.

The AFU+Si composition is therefore an injectable solution comprising crosslinked sodium hyaluronate with a molecular weight of 1.2 MDa and a final concentration of 2.5% by weight. This solution contains the organosilicon compound at a final concentration of 0.055% (equivalent amount of silicon) and 0.3 % by weight of lidocaine hydrochloride as an anesthetic agent

### Example 1 - protective effect against UV irradiation (hyaluronate)

The AFU+Si composition was tested to evaluate the protective effect against UV irradiation for sodium hyaluronate (NaHA) degradation in a model of human skin maintained in ex vivo survival, when compared to AFU.

Skin fragments from two subjects (skin 1: abdominoplasty, 39-year old male; skin 2: abdominoplasty, 45-year old female) were cut into 1 cm² pieces and washed three times using an antibiotic solution. Subcutaneous fat and lower dermis were mechanically removed using a surgical scalpel. One skin fragment was frozen at day 0 to assess the basal hyaluronan level.

Intradermal injections of AFU and AFU+Si were performed at day 0 (3 impacts of 20 µL), in the superficial dermis and the upper part of the middle dermis.

The skin biopsies were placed with the epithelium uppermost, at an air/liquid interface, on culture inserts in wells (filter pore size 12 µm, 1 cm² surface; Costar, VWR International).

Medium was added to the wells so that the surface of the medium was levelled with the filters. The medium was Dulbecco's minimal essential medium (Invitrogen Corporation), containing antibiotics, L-glutamine, bovine pituitary extract, growth factors and fetal calf serum (DAP). Passage took place by slow diffusion between the two compartments via the porous membrane. The medium was changed every 3 days.

The inserts were set on a twelve-well plate for 16 days at 37°C in a humidified atmosphere containing 5% CO₂, in an incubator.

To obtain aging of the skin with alteration of the dermis, UVA and UVB irradiation was used, which is known to induce alterations in the middle and deep dermis. The source of ultraviolet irradiation was a Vilber Lourmat simulator fitted out with a UVA irradiation source (365 nm) composed of Vilber Lourmat tubes T20.L365 mercury vapor tubes, low pressure, hot cathodes with a Vilber Lourmat RMX-365/312 radiometer. The UVB irradiation source (312 nm) was composed of Vilber Lourmat tubes T20.L312 mercury vapor tubes. A radiometer was associated with a microprocessor programmable in energy (J/cm²).

The doses applied were 8 J/cm² UVA and 2 J/cm² UVB at day 1 and day 3.

The three following conditions were compared:
- skin + UV treatment (control);
- skin injected with AFU + UV treatment (comparative);
- skin injected with AFU+Si + UV treatment (invention).

Samples of the skin fragments were taken every 4 days after UV aging at day 4, day 8 and day 12, to assess hyaluronan content by an ELISA assay.

Each skin fragment was lysed in a buffer (Cell lysis Buffer, Biotechne). The amount of hyaluronic acid was then measured by an immunoassay technique (ELISA Human Hyaluronan assay, Biotechne).

To compare the different results, the quantity of hyaluronic acid was related to the quantity of total proteins in the sample. The protein concentration (µg/ml) was spectrophotometrically determined at 562 nm (BCA assay, Pierce). The final results was expressed in ng/mg of protein.

The quantity of hyaluronic acid measured after the injection of products containing hyaluronic acid corresponds to the quantity of hyaluronic acid contained in the skin plus the hyaluronic acid contained in the injected product. After application of the UV stress, a decrease in hyaluronan content is observed at day 4 (2723,55 ng/mg) and at day 8 (2476,95 ng/mg) in comparison with hyaluronan at day 0 (3505,95 ng/mg) in the control samples. At day 12, an increase is observed (3474,3 ng/mg) corresponding to the natural repair of hyaluronan.

Over time, the quantity of hyaluronic acid dosed is always higher after injection of AFU + Si than after injection of AFU, meaning that the injection of the silicon compound protects hyaluronan from degradation.

The full results are presented in table 1 below:

**Table 1 - Hyaluronic acid measurement (in ng/mg)**

| | Mean ± SD |
|---|---|
| Day 0 | 3505.9 ± 869.6 |
| Day 4 (UV, control) | 2723.5 ± 790.1 |
| Day 4 (UV, AFU) | 3286.5 ± 1017.6 |
| Day 4 (UV, AFU+Si) | 3791.7 ± 1160.3 |
| Day 8 (UV, control) | 2476.9 ± 134.7 |
| Day 8 (UV, AFU) | 3641.9 ± 79.4 |
| Day 8 (UV, AFU+Si) | 4365.2 ± 504.2 |
| Day 12 (UV, control) | 3474.3 ± 337.7 |
| Day 12 (UV, AFU) | 3345.8 ± 153.9 |
| Day 12 (UV, AFU+Si) | 4790.1 ± 892.9 |

### Example 2 - protective effect against UV irradiation (collagen)

The compositions Si, AFU and AFU + Si were tested to evaluate the antiaging effect in a model of human skin maintained in ex vivo survival, with and without UV irradiation, through the quantification of collagen.

Fragments of normal human skin from 6 different donors (breast reduction or abdominoplasty) were obtained by plastic surgery and dissected into 3 cm² pieces by removing the hypodermis.

On day 0, the injections of the compositions were performed intradermally (9 impacts of 20 µL, in the superficial dermis and the upper part of the middle dermis.

To assess the effect of the injections in a UV oxidative stress model, a set of skins was injected after UV irradiation.

The following conditions were therefore tested:
- Control: no injection, no UV treatment;
- AFU: injection of AFU, no UV treatment;
- AFU+Si: injection of AFU+Si, no UV treatment;
- UV: no injection, UV treatment;
- UV+AFU: injection of AFU, UV treatment;
- UV+Si: injection of Conjonctyl, UV treatment;
- UV+AFU+Si: injection of AFU+Si, UV treatment.

The UV treatment consisted in irradiation with 8 J/cm² UVA and 4 J/cm² UVB after the injections (on day 0), with the same setup as in Example 1.

The skin fragments were deposited in inserts having a diameter of 3 cm, positioned on culture wells. Culture medium ensuring the survival of the skin was added to the bottom of the wells, and a passage by slow diffusion between the two compartments was ensured via a porous membrane (12 µm pore size). Environmental changes were carried out 3 times a week. The skin fragments were kept in survival for 14 days and then collected to analyze the synthesis of collagen.

After the 14 days of survival, some of the skin fragments were weighed and then digested enzymatically overnight at +4°C in a solution of acetic acid at 0.5 M containing pepsin. This method makes it possible to recover the newly synthesized collagen. After grinding with a potter, the amount of collagen (µg/ml) was evaluated by a spectrocolorimetric assay method at 540 nm: the acid-soluble collagen was detected after specific fixation of the red dye Sirius (Sircol Collagen Assay, Interchim). The results are expressed in µg of collagen per mg of tissue.

**Table 2 - Collagen measurement (in µg/mg)**

| Control | AFU | AFU+Si | UV | UV+ Si | UV+ AFU | UV+ AFU+Si |
|---|---|---|---|---|---|---|
| 14.13 | 14.25 | 15.34 | 11.73 | 12.97 | 12.97 | 14.94 |

In the UV aging model, a 17% decrease in collagen synthesis was observed compared to the control skin. After injection of AFU+Si (treatment according to the invention), an increase in collagen synthesis of 27.4% was demonstrated compared to the UV model (vs. 10.6% for the comparative Si treatment and AFU treatment).

### Example 3 - protective effect against UV irradiation (elastin)

The compositions AFU and AFU + Si were tested to evaluate the antiaging effect in a model of human skin maintained in ex vivo survival, with and without UV irradiation, through the quantification of elastin, in comparison with another independent study where a formulation of non-crosslinked hyaluronic acid including Si was tested in the same conditions.

The general protocol was similar to that of Example 2.

The following conditions were tested:
- Control: no injection, no UV treatment;
- AFU: injection of AFU, no UV treatment;
- AFU+Si: injection of AFU+Si, no UV treatment;UV: no injection, UV treatment;
- UV+AFU: injection of AFU, UV treatment;
- UV+AFU+Si: injection of AFU+Si, UV treatment.

After the 14 days of survival, some of the skin fragments were weighed and then solubilized by oxalic acid at 0.25 M at 100°C to obtain soluble fragments of alpha elastin polypeptides. After centrifugation to remove undigested tissue residues, the amount of soluble elastin was measured by a spectrocolorimetric assay method at 513 nm, after fixation of the dye 5,10,15,20-tetraphenyl-21,23-porphirin tetrasulfonate (Fastin Elastin Assay kit, Interchim). The results are expressed in µg of elastin per mg of tissue.

Average values were calculated on the 6 donors.

**Table 3 - Elastin measurement (in µg/mg)**

| Control | AFU | AFU+ Si | UV | UV + AFU | UV+ AFU+Si |
|---|---|---|---|---|---|
| 12.72 | 13.27 | 13.57 | 8.91 | 10.34 | 13.09 |

In the absence of UV exposure, injections of AFU and AFU+Si versus Si tend to slightly increase elastin synthesis in the dermis compared to control skin.

In the UV aging model, a 29.9% decrease in elastin synthesis was observed compared to control skin. After injection of AFU and AFU+Si an increase in elastin synthesis of 16% and 46.8% respectively was observed compared to the UV model.

Finally, in another, similar experiment carried out with an injection of MHA30 (same dosage as AFU+Si), an increase in elastin synthesis of 30% only was observed compared to the UV model. In particular, the following conditions were tested:
- Control: no injection, no UV treatment;
- MHA30: Injection of MHA30, no UV treatment;
- UV treatment;
- UV+MHA30: injection of MHA30, UV treatment;

Conditions of the test are the same as in Example 3: After the 14 days of survival, some of the skin fragments were weighed and then solubilized by oxalic acid at 0.25 M at 100°C to obtain soluble fragments of alpha elastin polypeptides. After centrifugation to remove undigested tissue residues, the amount of soluble elastin was measured by a spectrocolorimetric assay method at 513 nm, after fixation of the dye 5,10,15,20-tetraphenyl-21,23-porphirin tetrasulfonate (Fastin Elastin Assay kit, Interchim). The results are expressed in µg of elastin per mg of tissue.
Average values were calculated on the 6 donors.
The results obtained are presented in the below table

| | Control | MHA30 (No UV) | UV | MHA30 (UV treatment) |
|---|---|---|---|---|
| Elastin measurement (in µg/mg) | 4.96 | 5.23 | 3.54 | 4.62 |

For the same conditions, association of Si and crosslinked hyaluronic acid increase significatively elastin synthesis vs association of Si and uncrosslinked hyaluronic acid.

### Example 4 - tensor effect on fibroblasts

AFU+Si was tested to evaluate the effect on contractile force of fibroblasts in a Glassbox system, when compared to Si and AFU.

An equivalent dermis was developed in a culture box consisting of eight rectangular tanks. In each tank, two flexible blades were plunged, the lower parts of which were made up of grids on which the equivalent dermis clang during its polymerization. The equivalent dermis developed between the two blades to result in a rectangular shape slightly narrowed in the center.

Under the influence of the contraction forces developed by fibroblasts, the blades were deformed. Their deformation was measured in real time using optical fibers and adapted software. The deformation was proportional to the force developed within the equivalent dermis.

TGFβ1 at a concentration of 2.5 ng/ml was used as a positive contraction control, and as expected results in a significant increase in contractile forces.

Prior to the test for contractile force, different concentrations of products diluted in cellular culture medium were tested for cellular viability. Among these, the following concentrations were chosen for the contractile force test: 0.005%, 0.05%, 0.0005% and 0.0025%.

An increase in contractile forces was observed in the presence of Si at only one of the tested concentrations, namely 0.05%.

An increase in contractile forces was observed in the presence of AFU at two tested concentrations, namely 0.05% and 0.005%. An increase in contractile forces was observed in the presence of AFU+Si at the two lowest concentrations tested, namely 0.0005% and 0.0025%.

As a result, AFU+Si had a higher tensor effect on fibroblasts than Si and AFU, even at a low concentration.

### Example 5 - effect on the dimensions of fibroblast fibers

AFU+Si was tested to evaluate the role of protection for fibroblasts fibers (in particular the quality of fibers) after UV ageing when compared to AFU
Ex vivo skin explants kept in culture for 3 weeks with exposition to chronic UVB irradiation every 2 days were treated with AFU+Si and AFU. These skin cultures were stopped at different times (0, 45 minutes, 2 weeks and 3 weeks)
The structure and shape of the collagen fibers of 3 distinct areas of the papillary dermis was observed by transmission electron microscopy and measurements of the collagen fiber diameter was performed. It was observed that AFU+Si had a positive effect on the dimension of the fibers (augmented diameter of collagen fibers) compared to AFU at three weeks, which implies that the quality of collagen fibers is improved on the long term.

## Claims

1. A composition, comprising:
- an organosilicon compound of formula (I):
RₓSi(OH)₄₋ₓ (I)
or a salt thereof, wherein each R is independently a group containing from 1 to 20 carbon atoms, x = 1 to 3, and
- a crosslinked glycosaminoglycan or a salt thereof.

2. The composition of claim 1, in the form of an injectable solution.

3. The composition of any one of claims 1 to 2, having a pH from 5.0 to 7.0, preferably from 5.5 to 6.5.

4. The composition of any one of claims 1 to 3, wherein the glycosaminoglycan is selected from hyaluronic acid or a salt thereof, chondroitin sulfate and keratan sulfate, and is preferably hyaluronic acid or sodium hyaluronate.

5. The composition of any one of claims 1 to 4, wherein the molecular weight of the glycosaminoglycan prior to crosslinking ranges from 0.8 to 4.0 MDa, in particular from 1.0 to 2.0 MDa

6. The composition of any one of claims 1 to 5, wherein the organosilicon compound is monomethylsilanetriol or dimethylsilanediol or a salt thereof.

7. The composition of any of claims 1 to 6, wherein the organosilicon compound of formula (I) is present in an amount from 0.1% to 5%, in particular from 0.2% to 1%, by weight, relative to the total weight of the composition.

8. The composition of any of claims 1 to 7, wherein the crosslinked glycosaminoglycan is present in an amount from 0.5% to 5.0%, in particular from 1.5% to 2.5%, by weight, relative to the total weight of the composition.

9. The composition of any of claims 1 to 8, wherein the weight ratio of the organosilicon compound relative to the crosslinked glycosaminoglycan ranges from 1 to 50%, in particular from 5 to 20%.

10. The composition of any of claims 1 to 9, further comprising an anesthetic agent, preferably lidocaine, or a salt thereof.

11. The composition of claim 10, wherein the anesthetic agent is present in an amount from 0.1% to 5%, in particular from 0.2% to 0.4%, by weight, relative to the total weight of the composition.

12. The composition of any of claims 1 to 11, which is a cosmetic composition.

13. A method of cosmetic treatment of the skin, preferably for treating wrinkles or other dermal depressions and/or for improving the condition of the skin, notably for reducing signs of aging, the method comprising a step of administering the composition of claim 12 to a subject.

14. The composition of any of claims 1 to 11, which is a medicament for use in the *in situ* treatment of damaged dermis.

15. The composition of claim 14, wherein a dose comprised between 0.05 and 1.0 mg of said organosilicon compound is administered *in situ* per cm² of treated dermis.

## Patentansprüche

1. Zusammensetzung, umfassend:
- eine Organosiliciumverbindung der Formel (I):
RₓSi (OH)_{4-X} (I)
oder ein Salz davon, wobei jedes R unabhängig eine Gruppe mit 1 bis 20 Kohlenstoffatomen ist, x = 1 bis 3, und
- ein vernetztes Glykosaminoglykan oder ein Salz davon.

2. Zusammensetzung nach Anspruch 1 in Form einer injizierbaren Lösung.

3. Zusammensetzung nach einem der Ansprüche 1 bis 2, mit einem pH-Wert von 5,0 bis 7,0, vorzugsweise von 5,5 bis 6,5.

4. Zusammensetzung nach einem der Ansprüche 1 bis 3, wobei das Glykosaminoglykan ausgewählt ist aus Hyaluronsäure oder einem Salz davon, Chondroitinsulfat und Keratansulfat, und vorzugsweise Hyaluronsäure oder Natriumhyaluronat ist.

5. Zusammensetzung nach einem der Ansprüche 1 bis 4, wobei das Molekulargewicht des Glykosaminoglykans vor der Vernetzung im Bereich von 0,8 bis 4,0 MDa liegt, insbesondere von 1,0 bis 2,0 MDa.

6. Zusammensetzung nach einem der Ansprüche 1 bis 5, wobei die Organosiliciumverbindung Monomethylsilantriol oder Dimethylsilandiol oder ein Salz davon ist.

7. Zusammensetzung nach einem der Ansprüche 1 bis 6, wobei die Organosiliciumverbindung der Formel (I) in einer Menge von 0,1 % bis 5 %, insbesondere von 0,2 % bis 1 %, bezogen auf das Gesamtgewicht der Zusammensetzung, vorliegt.

8. Zusammensetzung nach einem der Ansprüche 1 bis 7, wobei das vernetzte Glykosaminoglykan in einer Menge von 0,5 % bis 5,0 %, insbesondere von 1,5 % bis 2,5 %, bezogen auf das Gesamtgewicht der Zusammensetzung, vorliegt.

9. Zusammensetzung nach einem der Ansprüche 1 bis 8, wobei das Gewichtsverhältnis der Organosiliciumverbindung zum vernetzten Glykosaminoglykan im Bereich von 1 bis 50 %, insbesondere von 5 bis 20 %, liegt.

10. Zusammensetzung nach einem der Ansprüche 1 bis 9, ferner umfassend ein Anästhetikum, vorzugsweise Lidocain, oder ein Salz davon.

11. Zusammensetzung nach Anspruch 10, wobei das Anästhetikum in einer Menge von 0,1 % bis 5 %, insbesondere von 0,2 % bis 0,4 %, bezogen auf das Gesamtgewicht der Zusammensetzung, vorliegt.

12. Zusammensetzung nach einem der Ansprüche 1 bis 11, die eine kosmetische Zusammensetzung ist.

13. Verfahren zur kosmetischen Behandlung der Haut, vorzugsweise zur Behandlung von Falten oder anderen dermalen Vertiefungen und/oder zur Verbesserung des Hautzustands, insbesondere zur Reduzierung von Alterserscheinungen, wobei das Verfahren einen Schritt des Verabreichens der Zusammensetzung nach Anspruch 12 an ein Subjekt umfasst.

14. Zusammensetzung nach einem der Ansprüche 1 bis 11, die ein Arzneimittel zur Verwendung bei der in situ Behandlung von geschädigter Dermis ist.

15. Zusammensetzung nach Anspruch 14, wobei eine Dosis von 0,05 bis 1,0 mg der genannten Organosiliciumverbindung in situ pro cm² behandelter Dermis verabreicht wird.

## Revendications

1. Composition, comprenant :
- un composé organosilicié de formule (I) :
RₓSi(OH)₄₋ₓ (I)
ou un sel de celui-ci, dans laquelle chaque R est indépendamment un groupe contenant de 1 à 20 atomes de carbone, x = 1 à 3, et
- un glycosaminoglycane réticulé ou un sel de celui-ci.

2. Composition selon la revendication 1, sous la forme d'une solution injectable.

3. Composition selon l'une quelconque des revendications 1 à 2, ayant un pH de 5,0 à 7,0, de préférence de 5,5 à 6,5.

4. Composition selon l'une quelconque des revendications 1 à 3, dans laquelle le glycosaminoglycane est sélectionné parmi l'acide hyaluronique ou un sel de celui-ci, le sulfate de chondroïtine et le sulfate de kératane, et est de préférence de l'acide hyaluronique ou du hyaluronate de sodium.

5. Composition selon l'une quelconque des revendications 1 à 4, dans laquelle le poids moléculaire du glycosaminoglycane avant réticulation va de 0,8 à 4,0 MDa, en particulier de 1,0 à 2,0 MDa

6. Composition selon l'une quelconque des revendications 1 à 5, dans laquelle le composé organosilicié est du monométhylsilanetriol ou du diméthylsilanediol ou un sel de celui-ci.

7. Composition selon l'une quelconque des revendications 1 à 6, dans laquelle le composé organosilicié de formule (I) est présent en une quantité de 0,1 % à 5 %, en particulier de 0,2 % à 1 %, en poids, par rapport au poids total de la composition.

8. Composition selon l'une quelconque des revendications 1 à 7, dans laquelle le glycosaminoglycane réticulé est présent en une quantité de 0,5 % à 5,0 %, en particulier de 1,5 % à 2,5 %, en poids, par rapport au poids total de la composition.

9. Composition selon l'une quelconque des revendications 1 à 8, dans laquelle le rapport pondéral du composé organosilicié par rapport au glycosaminoglycane réticulé va de 1 à 50 %, en particulier de 5 à 20 %.

10. Composition selon l'une quelconque des revendications 1 à 9, comprenant en outre un agent anesthésique, de préférence de la lidocaïne, ou un sel de celui-ci.

11. Composition selon la revendication 10, dans laquelle l'agent anesthésique est présent en une quantité de 0,1 % à 5 %, en particulier de 0,2 % à 0,4 %, en poids, par rapport au poids total de la composition.

12. Composition selon l'une quelconque des revendications 1 à 11, qui est une composition cosmétique.

13. Méthode de traitement cosmétique de la peau, de préférence pour traiter des rides ou autres dépressions dermiques et/ou pour améliorer l'état de la peau, notamment pour réduire des signes de vieillissement, la méthode comprenant une étape d'administration de la composition selon la revendication 12 à un sujet.

14. Composition selon l'une quelconque des revendications 1 à 11, qui est un médicament pour une utilisation dans le traitement *in situ* du derme endommagé.

15. Composition selon la revendication 14, dans laquelle une dose comprise entre 0,05 et 1,0 mg dudit composé organosilicié est administrée *in situ* par cm² de derme traité.
